Europäisches Patentamt

European Patent Office

Office européen des brevets

(19)

(11) Veröffentlichungsnummer: **0 248 252**
**A2**

(12) **EUROPÄISCHE PATENTANMELDUNG**

(21) Anmeldenummer: **87107151.0**

(22) Anmeldetag: **18.05.87**

(51) Int. Cl.³: **C 08 B 15/02**
**A 61 K 9/20**

(30) Priorität: **31.05.86 DE 3618377**

(43) Veröffentlichungstag der Anmeldung:
**09.12.87 Patentblatt 87/50**

(84) Benannte Vertragsstaaten:
**AT BE CH DE FR GB IT LI NL**

(71) Anmelder: **Wolff Walsrode Aktiengesellschaft**
**Postfach**
**D-3030 Walsrode 1(DE)**

(72) Erfinder: **Brauer, Oke, Dr.**
**Brüggemannstrasse 18**
**D 3030 Walsrode(DE)**

(72) Erfinder: **Szablikowski, Klaus, Dr.**
**Claudiusstrasse 3**
**D 3030 Walsrode(DE)**

(72) Erfinder: **Traenckner, Hans-Joachim, Dr.**
**Wedelstrasse 46**
**D 4150 Krefeld 1(DE)**

(74) Vertreter: **Zobel, Manfred, Dr. et al,**
**c/o BAYER AG K-RP Patentabteilung Q 18**
**D-5090 Leverkusen-Bayerwerk(DE)**

(54) **Verfahren zur Herstellung von mikrokristalliner Cellulose.**

(57) Verfahren zur Herstellung von mikrokristalliner Cellulose, dadurch gekennzeichnet, daß Cellulose mit einem Wassergehalt von 0,1 – 15 Gew.-% mit einem mit Wasser sauer reagierenden Gas bei einer Temperatur von 20 – 100°C behandelt wird.

EP 0 248 252 A2

Wolff Walsrode AG        3030 Walsrode-Bomlitz

Zb/bo/c

# Verfahren zur Herstellung von mikrokristalliner Cellulose

Gegenstand der Erfindung sind Verfahren zur Herstellung mikrokristalliner Cellulose, indem man die Cellulose mit sauer reagierenden Gasen behandelt und Cellulose, die nach dem erfindungsgemäßen Verfahren erhältlich ist.

Es ist bereits bekannt, mikrokristalline Cellulose herzustellen, indem man native oder regenerierte Cellulose oder Cellulosederivate einer Hydrolyse mit wäßrigen Lösungen von Mineralsäuren unterwirft. Dabei werden je nach Art und Intensität der Behandlung mehr oder weniger "amorphe" Teile der Cellulose entfernt, so daß unterschiedlich große Teile hoher Kristallinität zurückbleiben.

So wird in der DE-PS 744 595 ein Verfahren zur Herstellung von Celluosepulver beschrieben, indem man gebleichten Sulfitzellstoff mit wäßriger Salzsäure behandelt.

In der US-PS 2 978 446 wird erstmals die Dispersionsstabilität von mikrokristalliner Cellulose beschrieben, die erzielt wird, wenn Zellstoff mit Mineralsäure, bevorzugt 2,5 n Salzsäure, erhitzt wird.

WW 5258-EP

0248252

Weiterhin wird in der DE-OS 2 313 596 die Herstellung mikrokristalliner Cellulose durch Behandlung von Zellstoff mit wäßrigem Schwefeldioxid mit einem $SO_2$-Gehalt von 0,1- 10 Gew.-%, bezogen auf eingesetzte Wassermenge, bei einem Flottenverhältnis von 1:6 bis 1:15 gelehrt.

Die bekannten Verfahren arbeiten mit großen Überschüssen an wäßrigen Mineralsäuren. Dabei muß die als Filtrat anfallende Säure gereinigt und für den Wiedereinsatz aufkonzentriert oder andersweitig entsorgt werden. Außerdem müssen die entsprechenden Reaktionsgefäße aus korrosionsbeständigen Werkstoffen bestehen.

Auch wenn der Abbau der Cellulose mit Hilfe von wäßriger $SO_2$-Lösung erfolgt, muß mit einem großen Überschuß der Lösung gearbeitet werden, da bei zu geringer Wasserzugabe zu der abzubauenden Cellulose diese leicht verklumpt und der Abbau nicht gleichmäßig erfolgen kann.

Um so überraschender war es daher, daß es mit dem erfindungsgemäßen Verfahren gelingt, qualitativ hochwertige mikrokristalline Cellulose herzustellen, indem man auf eine nur wenig befeuchtete Cellulose nur geringe Mengen sauer reagierender Gase einwirken läßt.

Gegenstand der Erfindung sind daher Verfahren zur Herstellung von mikrokristalliner Cellulose, die dadurch gekennzeichnet sind, daß vorzugsweise feingemahlene, gereinigte, native oder regenerierte Cellulose, vorzugsweise Zellstoff mit einem Gehalt an α-Cellulose $\geq$80 Gew.-

WW 5258

%, mit einem $H_2O$-Gehalt von 0,1-15, vorzugsweise 2-6 Gew.-% mit einem mit Wasser sauer reagierenden Gas bei 20 - 100°C behandelt wird.

Bevorzugt sind Verfahren, bei denen mit dem Gas in einem Mol-Verhältnis von Cellulose: Gas von 1 : 0.005 bis 1 : 0.06, vorzugsweise 1 : 0.03, bei einer Temperatur von 20 - 100°C, vorzugsweise 75 - 90°C behandelt und, gegebenenfalls nach Neutralisation, das Reaktionsprodukt gebleicht, gewaschen, getrocknet und auf die gewünschte Feinheit gemahlen wird.

In einer anderen bevorzugten Ausführungsform wird die Cellulose, bezogen auf atro-Cellulose, mit 0,1-5 Gew.-%, vorzugsweise 0,2-2,5 Gew.-% bezogen auf atro-Cellulose, eines mit Wasser sauer reagierenden Gas unter einem Druck von 0,5-3, vorzugsweise 1-2 bar, und bei Temperaturen von 50-100, vorzugsweise 80-90°C behandelt, und gegebenenfalls nach Neutralisation das rieselfähige Reaktionsprodukt ausgewaschen und getrocknet wird.

Gegenstand der Erfindung ist weiterhin mikrokristalline Cellulose, die nach dem erfindungsgemäßen Verfahren erhältlich ist.

Als Gase für das erfindungsgemäße Verfahren können mit Wasser sauer reagierende Base vorzugsweise Chlorwasserstoff, Schwefeldioxid, Schwefeltrioxid und Stickstoffdioxid eingesetzt werden. Vorzugsweise wird HCl verwendet. Die Gase sollten in wasserfreier Form, wie sie allgemein in Druckbehältern im Handel erhältlich sind, eingesetzt werden. Die für die Abbaureaktion notwendige Menge an gasförmiger Verbindung wird vorzugsweise durch eine volumetrische oder gravimetrische Mengenmessung dosiert. Flüssige Mineralsäure bildet sich erst, wenn das Gas mit der in der gereinigten Cellulose stets vorhandenen Restfeuchte in Kontakt kommt.

Die als Ausgangsmaterial verwendete gereinigte Cellulose, vorzugsweise mit einem Gehalt von $\geq 80$ Gew.-% $\alpha$-Cellulose, ist vorzugsweise Zellstoff, der aus Nadelholz oder Buchenholz oder Baumwoll-Linters gewonnen wurde und allgemein auch zur Herstellung wasserlöslicher Celluloseether oder thermoplastischer Celluloseester verwendet wird. Darüber hinaus kann Regeneratcellulose oder von Weichmacher befreites Zellglas eingesetzt werden.

Der Zellstoff wird vorzugsweise durch Mahlen, Granulieren o. ä. vorzerkleinert und mit einer Feuchte eingesetzt, die im Bereich der Gleichgewichtsfeuchte von Cellulose liegt.

Die für die Durchführung des erfindungsgemäßen Verfahrens notwendigen Apparate sind bekannt; beispielsweise kann die Abbaureaktion, eventuell mit der nachfolgenden Neutralisation, in einem bewegten Trockner, Mischer oder in einer Schnecke durchgeführt werden. Es ist jedoch auch möglich, die Abbaureaktion in einem Festbett vorzunehmen. Wichtig ist jedoch, daß dadurch die geringe Menge Gas und damit Säure durch intensive Mischbewegung in dem Ausgangsmaterial gleichmäßig verteilt wird.

WW 5258

Am Ende der Reaktion kann das Restgas durch Anlegen von Unterdruck abgezogen werden, zusätzlich können sodann geringe Mengen nicht umgesetzten Reaktionsgases im Reaktionsgefäß durch Eintrag pulverförmiger Neutralisationsmittel wie Carbonaten oder Bicarbonaten oder auch lediglich durch Aufdüsen von gasförmigem oder in Wasser gelöstem Ammoniak neutralisiert werden. Die erhaltene Cellulose kann noch einer Bleichbehandlung unterworfen werden. Diese kann mit den üblichen Bleichchemikalien (Chlor, Hypochlorit, Chlorit, Peroxide u. ä.) durchgeführt werden.

Als vorteilhaft hat sich eine Behandlung mit einer wäßrigen Natriumchlorit-Lösung mit 0,03 Gew.-% $NaClO_2$ im sauren Milieu bei einer Temperatur von $90^0$ C erwiesen.

Die so erhaltene vorzugsweise gebleichte mikrokristalline Cellulose kann in einem Überschuß Wasser dispergiert werden, dabei hat sich der Einsatz von Trinkwasser mit 5 - $10^0$ dH Gesamthärte besonders bewährt. Anschließend wird abfiltriert oder abgeschleudert, wobei der Filterkuchen noch mit weiterem Wasser nachgewaschen werden kann. Dabei wird ein Produkt erhalten, das noch 30-60 Gew.-% Wasser enthält. Dieses Wasser wird in einem weiteren Trocknungsschritt entfernt. Dies kann durch Trocknen an der Luft oder aber in einem bekannten Trocknungsaggregat bei erhöhter Temperatur geschehen. Beispiele für Trockner sind Trockenschrank, Kanal-, Band-, Schaufel-, Mulden-, Riesel-, Trommel-, Röhren-, Mahl-, Taumel-, Zerstäubungs- (Sprüh), Strom- und Wirbelschichttrockner.

Wird die mikrokristalline Cellulose im Anschluß an den Trocknungsprozeß noch gemahlen, können Mühlen wie Stifts-, Strahl-, Schneid- und Scheibenmühlen eingesetzt werden.

Es ist auch möglich, die unzerkleinerte Zellstoffbahn in einem kontinuierlichen Verfahren in einer geeigneten Begasungskammer mit den gasförmigen Abbaureagenzien zu behandeln, anschließend nachzubehandeln, zu trocknen und dann erst zu zerkleinern.

Das erfindungsgemäße Verfahren hat u.a. auch den Vorteil, daß nur mit geringen Gasmengen gearbeitet werden muß und sich dementsprechend weit weniger Mineralsäure in Kontakt mit dem feuchten Zellstoff bildet. Damit werden aufwendige Entsorgungs- und Wiederaufbereitungsmaßnahmen überflüssig.

Die erfindungsgemäß hergestellte mikrokristalline Cellulose eignet sich hervorragend für die Herstellung von Tabletten als Füll-, Binde- und Sprengmittel, zur Naßregulation, aber auch als Füllmaterial bei der Abfüllung von Hartgelatinekapseln. Darüber hinaus kann sie als Adsorptionsmittel für analytische Trennverfahren z.B. in der Säulenchromatographie verwendet werden. Ebenso kann sie in der Lebensmittelindustrie, insbesondere bei kalorienreduzierten Nahrungsmitteln und in der Kosmetikindustrie als Bindemittel eingesetzt werden.

Die für den Einsatz von mikrokristalliner Cellulose als Tablettierhilfsmittel notwendigen Eigenschaften sind neben

WW 5258

der Sedimentationsstabilität in Wasser auch die Verpreßbarkeit zu Tabletten und Bruchfestigkeit sowie Auflösbarkeit daraus hergestellter Tabletten.

Die bei der Herstellung von Tabletten aufzuwendenden
Preßdrucke sollen zur Druckfestigkeit in einer linearen
Beziehung stehen. Die Abhängigkeit der Auflösbarkeit aus
mikrokristalliner Cellulose hergestellter Tabletten in
Wasser zu ihrer Härte, stellt dagegen eine exponentielle
Beziehung dar. Es ist üblich, diese als Kriterium für die
Eignung der mikrokristallinen Cellulose als Tablettierhilfsmittel heranzuziehen.

Die Sedimentationsstabilität der mikrokristallinen
Cellulose, die auch als ihr Identifikationsmerkmal gilt,
wird nach dem Deutschen Arzneibuch [DAB IX] bestimmt.

Diese Dispergierfähigkeit muß so gut sein, daß nach Dispergierung und entsprechender Standzeit der mikrokristallinen Cellulose keine Sedimentation unter Ausbildung einer
überstehenden Flüssigkeitsschicht eintritt. Damit besitzt
mikrokristalline Cellulose ein einfaches und eindeutiges
Unterscheidungsmerkmal gegenüber feingemahlenem Cellulose-
Pulver, welches in Wasser suspendiert, augenblicklich
absetzt und eine überstehende Wasserschicht ausbildet.

Die erfindungsgemäß hergestellte mikrokristalline
Cellulose erfüllt diese Bedingungen, so daß sie sich hervorragend als Tablettenhilfsmittel eignet.

WW 5258

Beispiel 1

1000 g feingemahlener Nadelholzzellstoff mit einer Feuchte von 3 Gew.-% werden in einen heizbaren 20 l-Labor-schüttelautoklav aus V4A-Stahl mit Innenemaillierung gefüllt. Anschließend wird der Autoklav zweimal bis zu einem Vakuum von 100 mbar evakuiert und das Vakuum jeweils mit Stickstoff gebrochen. Nachdem ein weiteres Mal evakuiert wurde, werden 5 g trockener Chlorwasserstoff durch Differenzwägung aus einem Druckgefäß eingeleitet. Nach Lösen der Druckschlauchverbindung wird der Laborautoklav unter schnellem Drehen innerhalb einer Stunde auf 80-85°C erhitzt und noch zwei Stunden bei 85°C rotiert. Anschließend wird abgekühlt, erneut evakuiert und das Vakuum gebrochen. Das Produkt wird auf einer Filternutsche mit 10 l Wasser säurefrei gewaschen und anschließend noch mit 300 ml 1 %iger Ammoniaklösung behandelt. Dann wird im Umlufttrockenschrank bei 110°C getrocknet und gegebenenfalls in einer Zentrifugalmühle bis zum notwendigen Korngrößenspektrum gemahlen.

Das Produkt erfüllt den Dispergiertest nach DAB IX. Die Grenzviskositätzahl ("intrinsic viscosity") der mikrokristallinen Cellulose beträgt 124 ml/g bestimmt in $Fe^{3+}$-Weinsäure-Natriumkomplex in verdünnter NaOH (Merkblatt IV 50/69 des Vereins der Zellstoff- und Papierchemiker- und Ingenieure).

Die Kenndaten sind in Tabelle 1 angegeben.

WW 5258

Tabelle 1

| | |
|---|---|
| Schüttgewicht (g/l) | 320 |
| NaCl-Gehalt (%) | 0,1 |
| Trockenverlust (%) | 3,5 |
| Siebanalyse Durchg. 0,1 mm | 94 % |
| Durchg. 0,063 mm | 69 % |

Aus dem Produkt wurden Tabletten unter Anwendung unterschiedlicher Preßdrucke hergestellt. Dabei ergab sich die geforderte lineare Beziehung zwischen Preßdruck und Druckfestigkeit der Tabletten (Tabelle 2).

Tabelle 2

| Preßdruck [MPa] | Druckfestigkeit [N] |
|---|---|
| 15 | 43 |
| 30 | 78 |
| 45 | 121 |
| 60 | 157 |

WW 5258

Beispiel 2

50 g feingemahlener Nadelholzzellstoff mit einer Feuchte von 5 Gew.-% werden in einen Glaslaborreaktor mit eingesetztem Rührwerk und Gaszu- und ableitungen gefüllt. Anschließend wird der Laborreaktor auf die Reaktionstemperatur von 80°C aufgeheizt und dann evakuiert. In den Reaktor werden aus einem kalibrierten Glaszylinder 200 ml Chlorwasserstoff-Gas eindosiert. Nach Schließen der Reaktorhähne läßt man 45 min. rühren. Danach wird auf Raumtemperatur gekühlt, Vakuum angelegt, um Restmengen an Gas zu entfernen, und danach belüftet.

Das gelbliche, pulverige Reaktionsprodukt wird dem Reaktor entnommen und wie folgt gebleicht: Zugabe der zehnfachen Menge 0.03 Gew.-%iger wäßriger Natriumchlorit-Lösung (bezogen auf die eingesetzte Cellulose-Menge), eingestellt auf pH 2, auf eine Temperatur von 90°C erhitzt. Die Bleichzeit beträgt 1 h. Danach wird der Bleichvorgang durch Zugabe von festem Natriumthiosulfat bis zu einem Farbumschlag von gelb nach weiß beendet. Die Suspension wird durch Zugabe von festem Natriumhydrogencarbonat neutralisiert und filtriert. Der Filterkuchen wird mit der 20 fachen Menge an Trinkwasser, bezogen auf das Feuchtgewicht, versetzt und gerührt. Anschließend wird abgesaugt und der Filterkuchen bei 80°C im Trockenschrank getrocknet.

Die Grenzviskositätszahl (Merkblatt IV/50/69 des Vereins der Zellstoff- und Papierchemiker und Ingenieure) be-trägt 320 ml/g. Das Produkt erfüllt den Dispergiertest nach DAB IX.

WW 5258

Patenansprüche

1. Verfahren zur Herstellung von mikrokristalliner Cellulose, dadurch gekennzeichnet, daß Cellulose mit einem Wassergehalt von 0,1 - 15 Gew.-% mit einem mit Wasser sauer reagierenden Gas bei einer Temperatur von 20 - 100°C behandelt wird.

2. Verfahren nach Anspruch 1, dadurch gekennzeichnet, daß, native oder regenerierte Cellulose eingesetzt wird.

3. Verfahren nach Anspruch 1, dadurch gekennzeichnet, daß als Cellulose Zellstoff mit einem Gehalt an α-Cellulose $\geq$ 80 Gew.-% eingesetzt wird.

4. Verfahren nach Anspruch 1, dadurch gekennzeichnet, daß als Gas HCl eingesetzt wird.

5. Verfahren nach Anspruch 1, dadurch gekennzeichnet, daß das Reaktionsprodukt gebleicht, gewaschen, getrocknet und gegebenenfalls gemahlen wird.

6) Verfahren nach Anspruch 1, dadurch gekennzeichnet, daß der Wassergehalt der Cellulose 2 - 6 Gew.-% beträgt.

7. Verfahren nach Anspruch 1, dadurch gekennzeichnet, daß das Molverhältnis Cellulose: sauer reagierendem

WW 5258

Gas 1 : 0.005 bis 1 : 0.06, beträgt.

8. Verfahren nach Anspruch 5, dadurch gekennzeichnet, daß mit Natriumchlorit gebleicht wird.

9. Verwendung der gemäß wenigstens einem der vorangehenden Ansprüche erhaltenen mikrokristallinen Cellulose als Tablettierhilfsmittel.

10. Mikrokristalline Cellulose erhältlich gemäß Anspruch 1.

WW 5258